# EUROPÄISCHE PATENTSCHRIFT

(11) **EP 1 086 096 B1**
(45) Veröffentlichungstag und Bekanntmachung des Hinweises auf die Patenterteilung: **23.07.2003**
(21) Anmeldenummer: 99926441.9
(22) Anmeldetag: 27.05.1999
(51) Int. Cl.: C07D 405/12, A61K 31/44, A61K 31/34, A61K 31/35, C07D 493/10, C07D 307/20, C07D 309/10

(54) **BENZAMIDE MIT TETRAHYDROFURANYLOXY-SUBSTITUENTEN ALS INHIBITOREN DER PHOSPHODIESTERASE 4**
BENZAMIDES WITH TETRAHYDROFURANYLOXY SUBSTITUTENTS AS PHOSPHODIESTERASE 4 INHIBITORS
BENZAMIDES AVEC DES SUBSTITUANTS TETRAHYDROFURANYLOXY UTILISES COMME INHIBITEURS DE LA PHOSPHODIESTERASE 4

(30) Priorität: 10.06.1998 EP 98110710
(43) Veröffentlichungstag der Anmeldung: 28.03.2001
(73) Patentinhaber: ALTANA Pharma AG, 78467 Konstanz (DE)
(72) Erfinder: MARTIN, Thomas, D-78462 Konstanz (DE); ULRICH, Wolf-Rüdiger, D-78464 Konstanz (DE); AMSCHLER, Hermann, / (DE); SCHMIDT, Beate, D-78476 Allensbach (DE); FLOCKERZI, Dieter, D-78476 Allensbach (DE); HATZELMANN, Armin, D-78467 Konstanz (DE); BOSS, Hildegard, D-78476 Allensbach (DE); BEUME, Rolf, D-78465 Konstanz (DE); KILIAN, Ulrich, D-78479 Reichenau (DE); KLEY, Hans-Peter, D-78476 Allensbach (DE); BÄR, Thomas, D-78479 Reichenau (DE)
(86) Internationale Anmeldenummer: EP9903669
(87) Internationale Veröffentlichungsnummer: WO99064414

(56) Entgegenhaltungen:
- WO-A-95/20578
- WO-A-96/03399
- WO-A-97/20833
- WO-A-98/07715

## Beschreibung

Die Erfindung betrifft neue Benzamide, die in der pharmazeutischen Industrie zur Herstellung von Medikamenten verwendet werden.

In den internationalen Patentanmeldungen WO93/25517, WO95/01338 und WO96/03399 werden trisubstituierte Phenylderivate als selektive PDE4-Hemmer offenbart. In den internationalen Patentanmeldungen WO94/02465 und WO95/20578 werden ebenfalls 3- oder mehrfach substituierte Phenylderivate als Inhibitoren der Phosphodiesterase 4 und der TNF-Sekretion beschrieben.

Es wurde nun gefunden, daß sich die nachfolgend näher beschriebenen Verbindungen, sie sich von den vorveröffentlichten Verbindungen durch eine andersartige Substitution unterscheiden, überraschende und besonders vorteilhafte Eigenschaften besitzen.

Gegenstand der Erfindung sind somit Verbindungen der Formel I, 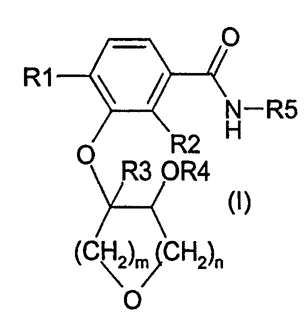 worin
- R1: 1-6C-Alkoxy, 3-7C-Cycloalkoxy, 3-7C-Cycloalkylmethoxy, Benzyloxy oder ganz oder überwiegend durch Fluor substituiertes 1-4C-Alkoxy bedeutet,
- R2: Wasserstoff und
- R3: Wasserstoff bedeutet
oder
- R2 und R3: gemeinsam eine Methylengruppe darstellen,
- R4: Wasserstoff, 1-8C-Alkyl, 1-6C-Alkoxy-1-4C-alkyl, 1-6C-Alkylthio-1-4-alkyl, 1-6C-Alkylsulfinyl-1-4C-alkyl, 1-6C-Alkylsulfonyl-1-4C-alkyl, 1-8C-Alkylcarbonyl, 3-7C-Cycloalkyl, 3-7C-Cycloalkylmethyl, Phenyl-1-4C-alkyl oder ganz oder überwiegend durch Fluor substituiertes 1-4C-Alkyl bedeutet,
- R5: Phenyl, Pyridyl, durch R51, R52 und R53 substituiertes Phenyl oder durch R54, R55, R56 und R57 substituiertes Pyridyl bedeutet, wobei
R51 Hydroxy, Halogen, Cyano, Carboxyl, Trifluormethyl, 1-4C-Alkyl, 1-4C-Alkoxy, 1-4C-Alkoxycarbonyl, 1-4C-Alkylcarbonyl, 1-4C-Alkylcarbonyloxy, Amino, Mono- oder Di-1-4C-alkylamino oder 1-4C-Alkylcarbonylamino,
R52 Wasserstoff, Hydroxy, Halogen, Amino, Trifluormethyl, 1-4C-Alkyl oder 1-4C-Alkoxy,
R53 Wasserstoff, Halogen, 1-4C-Alkyl oder 1-4C-Alkoxy,
R54 Hydroxy, Halogen, Cyano, Carboxyl, 1-4C-Alkyl, 1-4C-Alkoxy, 1-4C-Alkoxycarbonyl oder Amino,
R55 Wasserstoff, Halogen, Amino oder 1-4C-Alkyl,
R56 Wasserstoff oder Halogen und
R57 Wasserstoff oder Halogen bedeutet,
- n: für 1 oder 2 steht,
- m: für 1 oder 2 steht,
wobei die Summe aus m und n nur die Werte 2 oder 3 annehmen darf,
die Salze dieser Verbindungen sowie die N-Oxide der Pyridine und deren Salze.

1-8C-Alkyl steht für geradkettige oder verzweigte Alkylreste mit 1 bis 8 Kohlenstoffatomen. Beispielsweise seien genannt der Octyl-, Isooctyl- (6-Methylheptyl-), Heptyl-, Isoheptyl- (5-Methylhexyl-), Hexyl-, Isohexyl- (4-Methylpentyl-), Neohexyl- (3,3-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-6C-Alkoxy steht für einen Rest, der neben dem Sauerstoffatom einen geradkettigen oder verzweigten Alkylrest mit 1 bis 6 Kohlenstoffatomen enthält. Als Alkoxyreste mit 1 bis 6 Kohlenstoffatomen seien beispielsweise genannt der Hexyloxy-, Isohexyloxy- (4-Methylpentyloxy-), Neohexyloxy- (3,3-Dimethylbutoxy-), Pentyloxy-, Isopentyloxy- (3-Methylbutoxy-), Neopentyloxy- (2,2-Dimethylpropoxy-), Butoxy-, iso-Butoxy-, sec.-Butoxy-, tert.-Butoxy-, Propoxy-, Isopropoxy-, Ethoxy- und der Methoxyrest.

3-7C-Cycloalkoxy steht für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht für Cyclopropylmethoxy, Cyclobutylmethoxy, Cyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder überwiegend durch Fluor substituiertes 1-4C-Alkoxy seien beispielsweise der 2,2,3,3,3-Pentafluorpropoxy-, der Perfluorethoxy-, der 1,2,2-Trifluorethoxy-, insbesondere der 1,1,2,2-Tetrafluorethoxy-, der 2,2,2-Trifluorethoxy-, der Trifluormethoxy- und bevorzugt der Difluormethoxyrest genannt.

1-6C-Alkoxy-1-4C-alkyl steht für einen der oben definierten 1-4C-Alkylreste, der durch einen 1-6C-Alkoxyrest substituiert ist. Beispielhaft genannt seien der Methoxymethyl-, t-Butoxymethyl-, 1-Ethoxyethylund der 1-Methyl-1-methoxyethylrest.

1-6C-Alkylthio-1-4C-alkyl steht für einen 1-4C-Alkylrest, der durch einen 1-6C-Alkylthiorest substituiert ist. Beispielhaft genannt seien der Methylthiomethyl- und der tert.-Butylthiomethylrest.

1-6C-Alkylsulfinyl-1-4C-alkyl steht für einen 1-4C-Alkylrest, der durch einen 1-6C-Alkylsulfinylrest substituiert ist. Beispielhaft genannt sei der Methylsulfinylmethylrest.

1-6C-Alkylsulfonyl-1-4C-alkyl steht für einen 1-4C-Alkylrest, der durch einen 1-6C-Alkylsulfonylrest substituiert ist. Beispielhaft genannt sei der Methylsulfonylmethylrest.

1-8C-Alkylcarbonyl steht für einen Rest, der neben der Carbonylgruppe einen der vorstehend genannten 1-8C-Alkylreste enthält. Beispielsweise sei der Acetyl-, Propanoyl- und der Butanoylrest genannt.

3-7C-Cycloalkyl steht für Cyclopropyl, Cyclobutyl, Cyclopentyl, Cyclohexyl und Cycloheptyl, wovon Cyclopropyl, Cyclobutyl und Cyclopentyl bevorzugt sind.

3-7C-Cycloalkylmethyl steht für einen Methylrest, der durch einen der vorstehend genannten 3-7C-Cycloalkylreste substituiert ist. Bevorzugt seien die 3-5C-Cycloalkylmethylreste Cyclopropylmethyl, Cyclobutylmethyl und Cyclopentylmethyl genannt.

Phenyl-1-4C-alkyl steht für einen der oben genannten, durch Phenyl substituierten 1-4C-Alkylreste. Beispielsweise seien der Phenethyl- und der Benzylrest genannt.

Als ganz oder überwiegend durch Fluor substituiertes 1-4C-Alkyl seien beispielsweise der 2,2,3,3,3-Pentafluorpropyl-, der Perfluorethyl-, der 1,2,2-Trifluorethyl-, insbesondere der 1,1,2,2-Tetrafluorethyl-, der 2,2,2-Trifluorethyl-, der Trifluormethyl- und bevorzugt der Difluormethylrest genannt.

Halogen im Sinne der Erfindung ist Brom, Chlor und Fluor.

1-4C-Alkoxycarbonyl steht für eine Carbonylgruppe, an die einer der vorstehend genannten 1-4C-Alkoxyreste gebunden ist. Beispielsweise seien der Methoxycarbonyl- (CH₃O-C(O)-) und der Ethoxycarbonylrest (CH₃CH₂O-C(O)-) genannt.

1-4C-Alkylcarbonyloxyreste enthalten neben dem Sauerstoffatom einen der vorstehend genannten 1-4C-Alkylcarbonylreste. Beispielsweise sei der Acetoxyrest (CH₃C(O)O-) genannt.

Mono- oder Di-1-4C-alkylaminoreste enthalten neben dem Stickstoffatom einen bzw. zwei der vorstehend genannten 1-4C-Alkylreste. Beispielsweise sei der Dimethylaminorest genannt.

Als 1-4C-Alkylcarbonylaminorest sei beispielsweise der Propionylamino- (C₃H₇C(O)NH-) und der Acetylaminorest (CH₃C(O)NH-) genannt.

Als beispielhafte, durch R51, R52 und R53 substituierte Phenylreste seien die Reste 2-Acetylphenyl, 2-Aminophenyl, 2-Bromphenyl, 2-Chlorphenyl, 2,3-Dichlorphenyl, 2,4-Dichlorphenyl, 4-Diethylamino-2-methylphenyl, 4-Brom-2-trifluormethylphenyl, 2-Carboxy-5-chlorphenyl, 3,5-Dichlor-2-hydroxyphenyl, 2-Brom-4-carboxy-5-hydroxyphenyl, 2,6-Dichlorphenyl, 2,5-Dichlorphenyl, 2,4,6-Trichlorphenyl, 2,4,6-Trifluorphenyl, 2,6-Dibromphenyl, 2-Cyanphenyl, 4-Cyan-2-fluorphenyl, 2-Fluorphenyl, 2,4-Difluorphenyl, 2,6-Difluorphenyl, 2-Chlor-6-fluorphenyl, 2-Hydroxyphenyl, 2-Hydroxy-4-methoxyphenyl, 2,4-Dihydroxyphenyl, 2-Methoxyphenyl, 2,3-Dimethoxyphenyl, 2,4-Dimethoxyphenyl, 2,6-Dimethoxyphenyl, 2-Dimethylaminophenyl, 2-Methylphenyl, 2-Chlor-6-methylphenyl, 2,4-Dimethylphenyl, 2,6-Dimethylphenyl, 2,3-Dimethylphenyl, 2-Methoxycarbonylphenyl, 2-Trifluormethylphenyl, 2,6-Dichlor-4-methoxyphenyl, 2,6-Dichlor-4-cyanphenyl, 2,6-Dichlor-4-aminophenyl, 2,6-Dichlor-4-methoxycarbonylphenyl, 4-Acetylamino-2,6-dichlorphenyl und 2,6-Dichlor-4-ethoxycarbonylphenyl genannt.

Als beispielhafte, durch R54, R55, R56 und R57 substituierte Pyridylreste seien die Reste 3,5-Dichlorpyrid-4-yl, 2,6-Diaminopyrid-3-yl, 4-Aminopyrid-3-yl, 3-Methylpyrid-2-yl, 4-Methylpyrid-2-yl, 5-Hydroxypyrid-2-yl, 4-Chlorpyrid-3-yl, 3-Chlorpyrid-2-yl, 3-Chlorpyrid-4-yl, 2-Chlorpyrid-3-yl, 2,3,5,6-Tetrafluorpyrid-4-yl, 3,5-Dichlor-2,6-difluorpyrid-4-yl, 3,5-Dibrompyrid-2-yl, 3,5-Dibrompyrid-4-yl, 3,5-Dichlorpyrid-4-yl, 2,6-Dichlorpyrid-3-yl, 3,5-Dimethylpyrid-4-yl, 3-Chlor-2,5,6-trifluorpyrid-4-yl und 2,3,5-Trifluorpyrid-4-yl genannt.

Als Salze kommen für Verbindungen der Formel I - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali-(Lithium-, Natrium-, Kalium-) oder Calcium-, Aluminium-, Magnesium-, Titan-, Ammonium-, Megluminoder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Dem Fachmann ist bekannt, daß die erfindungsgemäßen Verbindungen als auch ihre Salze, wenn sie zum Beispiel in kristalliner Form isoliert werden, verschiedene Mengen an Lösungsmitteln enthalten können. Die Erfindung umfaßt daher auch alle Solvate und insbesondere alle Hydrate der Verbindungen der Formel 1, sowie alle Solvate und insbesondere alle Hydrate der Salze der Verbindungen der Formel I.

Hervorzuhebende Verbindungen der Formel I sind solche, in denen
- R1: 1-40-Alkoxy, 3-5C-Cycloalkoxy, 3-5C-Cycloalkylmethoxy oder ganz oder überwiegend durch Fluor substituiertes 1-4C-Alkoxy bedeutet,
- R2: Wasserstoff und
- R3: Wasserstoff bedeutet,
oder
- R2 und R3: gemeinsam eine Methylengruppe darstellen,
- R4: Wasserstoff, 1-6C-Alkyl, 1-4C-Alkoxy-1-4C-alkyl, 1-4C-Alkylthio-1-4C-alkyl, 1-6C-Alkylcarbonyl, 3-7C-Cycloalkyl, 3-7C-Cycloalkylmethyl, Phenyl-1-4C-alkyl oder ganz oder überwiegend durch Fluor substituiertes 1-4C-Alkyl bedeutet,
- R5: Phenyl, Pyridyl, durch R51, R52 und R53 substituiertes Phenyl oder durch R54, R55, R56 und R57 substituiertes Pyridyl bedeutet, wobei
R51 Halogen, Cyano, Carboxyl, 1-4C-Alkyl, 1-4C-Alkoxy oder 1-4C-Alkoxycarbonyl,
R52 Wasserstoff, Halogen, 1-4C-Alkyl oder 1-4C-Alkoxy,
R53 Wasserstoff, Halogen, 1-4C-Alkyl oder 1-4C-Alkoxy,
R54 Halogen oder 1-4C-Alkyl,
R55 Wasserstoff oder Halogen,
R56 Wasserstoff oder Halogen und
R57 Wasserstoff oder Halogen bedeutet,
- n: für 1 oder 2 steht,
- m: für 1 oder 2 steht,
wobei die Summe aus m und n nur die Werte 2 oder 3 annehmen darf,
die Salze dieser Verbindungen sowie die N-Oxide der Pyridine und deren Salze.

Besonders hervorzuhebende Verbindungen der Formel I sind solche, in denen
- R1: Methoxy oder Difluormethoxy bedeutet,
- R2: Wasserstoff und
- R3: Wasserstoff bedeutet
oder
- R2 und R3: gemeinsam eine Methylengruppe darstellen,
- R4: Wasserstoff, 1-6C-Alkyl, 1-4C-Alkoxy-1-4C-alkyl, 1-4C-Alkylthio-1-4C-alkyl, 1-4C-Alkylcarbonyl, Phenethyl oder Benzyl bedeutet,
- R5: 2-Bromphenyl, 2-Chlorphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2-Chlor-6-fluorphenyl, 2,6-Difluorphenyl, 2,6-Dichlorphenyl, 3,5-Dichlorpyrid-4-yl, 3-Methylpyrid-2-yl, 2-Chlorpyrid-3-yl, 3-Chlorpyrid-4-yl, 3,5-Dibrompyrid-2-yl, 3,5-Difluorpyrid-4-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
- n: für 1 steht,
- m: für 1 steht und
die Salze dieser Verbindungen sowie die N-Oxide der Pyridine und deren Salze.

Eine Ausgestaltung (Ausgestaltung a) der besonders hervorzuhebenden Verbindungen sind jene Verbindungen der Formel I, in denen
- R1: Methoxy oder Difluormethoxy bedeutet,
- R2: Wasserstoff und
- R3: Wasserstoff bedeutet,
- R4: Wasserstoff, 1-6C-Alkyl, 1-4C-Alkoxy-1-4C-alkyl, 1-4C-Alkylthio-1-4C-alkyl, 1-4C-Alkylcarbonyl, Phenethyl oder Benzyl bedeutet,
- R5: 2-Bromphenyl, 2-Chlorphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2-Chlor-6-fluorphenyl, 2,6-Difluorphenyl, 2,6-Dichlorphenyl, 3,5-Dichlorpyrid-4-yl, 3-Methylpyrid-2-yl, 2-Chlorpyrid-3-yl, 3-Chlorpyrid-4-yl, 3,5-Dibrompyrid-2-yl, 3,5-Difluorpyrid-4-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
- n: für 1 steht,
- m: für 1 steht und
die Salze dieser Verbindungen sowie die N-Oxide der Pyridine und deren Salze.

Eine weitere Ausgestaltung (Ausgestaltung b) der besonders hervorzuhebenden Verbindungen sind jene Verbindungen der Formel I, in denen
- R1: Methoxy oder Difluormethoxy bedeutet,
- R2 und R3: gemeinsam eine Methylengruppe darstellen,
- R4: Wasserstoff, 1-6C-Alkyl, 1-4C-Alkoxy-1-4C-alkyl, 1-4C-Alkylthio-1-4C-alkyl, 1-4C-Alkylcarbonyl, Phenethyl oder Benzyl bedeutet,
- R5: 2-Bromphenyl, 2-Chlorphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2-Chlor-6-fluorphenyl, 2,6-Difluorphenyl, 2,6-Dichlorphenyl, 3,5-Dichlorpyrid-4-yl, 3-Methylpyrid-2-yl, 2-Chlorpyrid-3-yl, 3-Chlorpyrid-4-yl, 3,5-Dibrompyrid-2-yl, 3,5-Difluorpyrid-4-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
- n: für 1 steht,
- m: für 1 steht und
die Salze dieser Verbindungen sowie die N-Oxide der Pyridine und deren Salze.

Bevorzugte Verbindungen der Formel I sind solche, in denen
- R1: Methoxy oder Difluormethoxy bedeutet,
- R2: Wasserstoff und
- R3: Wasserstoff bedeutet,
oder
- R2 und R3: gemeinsam eine Methylengruppe darstellen,
- R4: Wasserstoff, 1-4C-Alkyl, 1-2C-Alkoxy-1-2C-alkyl, 1-2C-Alkylthio-1-2C-alkyl, 1-4C-Alkylcarbonyl oder Benzyl bedeutet,
- R5: 3,5-Dichlorpyrid-4-yl, 2-Chlorpyrid-3-yl, 3-Chlorpyrid-4-yl, 3,5-Dibrompyrid-2-yl, 3,5-Difluorpyrid-4-yl, 2,3,5,6-Tetrafluorpyrid-4-yl, 3-Chlor-2,5,6-trlfluorpyrid-4-yl, 3,5-Dichlor-2,6-difluorpyrid-4-yl oder 2,6-Dichlorpyrid-3-yl bedeutet,
- n: für 1 steht,
- m: für 1 steht und
die Salze dieser Verbindungen sowie die N-Oxide der Pyridine und deren Salze.

Bevorzugte Verbindungen der Formel I der Ausgestaltung a sind solche, in denen
- R1: Methoxy oder Difluormethoxy bedeutet,
- R2: Wasserstoff und
- R3: Wasserstoff bedeutet,
- R4: Wasserstoff, 1-4C-Alkyl, 1-2C-Alkoxy-1-2C-alkyl, 1-2C-Alkylthio-1-2C-alkyl oder Benzyl bedeutet,
- R5: 3,5-Dichlorpyrid-4-yl, 2-Chlorpyrid-3-yl, 3-Chlorpyrid-4-yl, 3,5-Dibrompyrid-2-yl, 3,5-Difluorpyrid-4-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
- n: für 1 steht,
- m: für 1 steht und
die Salze dieser Verbindungen sowie die N-Oxide der Pyridine und deren Salze.

Bevorzugte Verbindungen der Formel I der Ausgestaltung b sind solche, in denen
- R1: Methoxy oder Difluormethoxy bedeutet,
- R2 und R3: gemeinsam eine Methylengruppe darstellen,
- R4: Wasserstoff, 1-4C-Alkyl oder Benzyl bedeutet,
- R5: 3,5-Dichlorpyrid-4-yl, 2-Chlorpyrid-3-yl, 3-Chlorpyrid-4-yl, 3,5-Dibrompyrid-2-yl, 3,5-Difluorpyrid-4-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
- n: für 1 steht,
- m: für 1 steht und
die Salze dieser Verbindungen sowie die N-Oxide der Pyridine und deren Salze.

Besonders bevorzugte Verbindungen der Formel I sind solche, in denen
- R1: Methoxy bedeutet,
- R2: Wasserstoff und
- R3: Wasserstoff bedeutet,
oder
- R2 und R3: gemeinsam eine Methylengruppe darstellen,
- R4: Wasserstoff, Methyl, Ethyl, Methylthiomethyl oder Benzyl bedeutet,
- R5: 3,5-Dichlorpyrid-4-yl bedeutet,
- n: für 1 steht,
- m: für 1 steht und
die Salze dieser Verbindungen.

Besonders bevorzugte Verbindungen der Formel I der Ausgestaltung a sind solche, in denen
- R1: Methoxy bedeutet,
- R2: Wasserstoff und
- R3: Wasserstoff bedeutet,
- R4: Wasserstoff, Methyl, Ethyl, Methylthiomethyl oder Benzyl bedeutet,
- R5: 3,5-Dichlorpyrid-4-yl bedeutet,
- n: für 1 steht,
- m: für 1 steht und
die Salze dieser Verbindungen.

Besonders bevorzugte Verbindungen der Formel I der Ausgestaltung b sind solche, in denen
- R1: Methoxy bedeutet,
- R2 und R3: gemeinsam eine Methylengruppe darstellen,
- R4: Methyl oder Benzyl bedeutet,
- R5: 3,5-Dichlorpyrid-4-yl bedeutet,
- n: für 1 steht,
- m: für 1 steht und
die Salze dieser Verbindungen.

Bei den Verbindungen der Formel I handelt es sich um chirale Verbindungen. Chiralitätszentren treten an den mit den Substituenten (Gruppen) R3 und OR4 verbundenen Kohlenstoffatomen auf.

Die Erfindung umfaßt sowohl die reinen Enantiomeren als auch deren Gemische in jedem Mischungsverhältnis, einschließlich der Racemate. Die Enantiomeren können in an sich bekannter Weise, beispielsweise durch Herstellung und Trennung entsprechender diastereomerer Verbindungen, separiert werden.

Bilden R2 und R3 gemeinsam eine Methylengruppe, so liegt eine Spiroverbindung vor.

Weiterer Gegenstand der Erfindung sind Verfahren zur Herstellung der Verbindungen der Formel I und ihrer Salze, sowie der N-Oxide der Pyridine und ihrer Salze.

Das erste Verfahren ist dadurch gekennzeichnet, daß man Verbindungen der Formel II 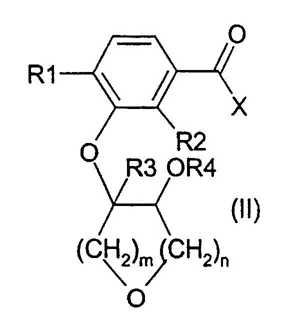 in denen R1, R2, R3, R4, m und n die oben angegebenen Bedeutungen haben und X eine geeignete Abgangsgruppe darstellt, mit Aminen R5-NH₂ umsetzt, und daß man gewünschtenfalls anschließend erhaltene Verbindungen der Formel I in ihre Salze und/oder erhaltene Pyridine in die N-Oxide und gewünschtenfalls anschließend in die Salze überführt, oder daß man gewünschtenfalls anschließend erhaltene Salze der Verbindungen der Formel I in die freien Verbindungen überführt.

Welche Abgangsgruppen X geeignet sind, ist dem Fachmann aufgrund seines Fachwissens geläufig. Beispielsweise wird von geeigneten Säurehalogeniden der Formel II (X=CI oder Br) ausgegangen. Im übrigen erfolgt die Umsetzung beispielsweise so wie in den nachfolgenden Beispielen beschrieben oder auf eine dem Fachmann an sich vertraute Weise (z.B. so, wie in den internationalen Patentanmeldungen WO 95/01338 oder WO96/0339 beschrieben).

Die N-Oxidation erfolgt auf eine dem Fachmann ebenfalls vertraute Weise, z.B. mit Hilfe von m-Chlorperoxibenzoesäure in Dichlormethan bei Raumtemperatur. Welche Reaktionsbedingungen für die Durchführung des Verfahrens im einzelnen erforderlich sind, ist dem Fachmann aufgrund seines Fachwissens geläufig.

Die Verbindungen der Formel II können gemäß dem allgemeinen Reaktionsschema 1 hergestellt werden.

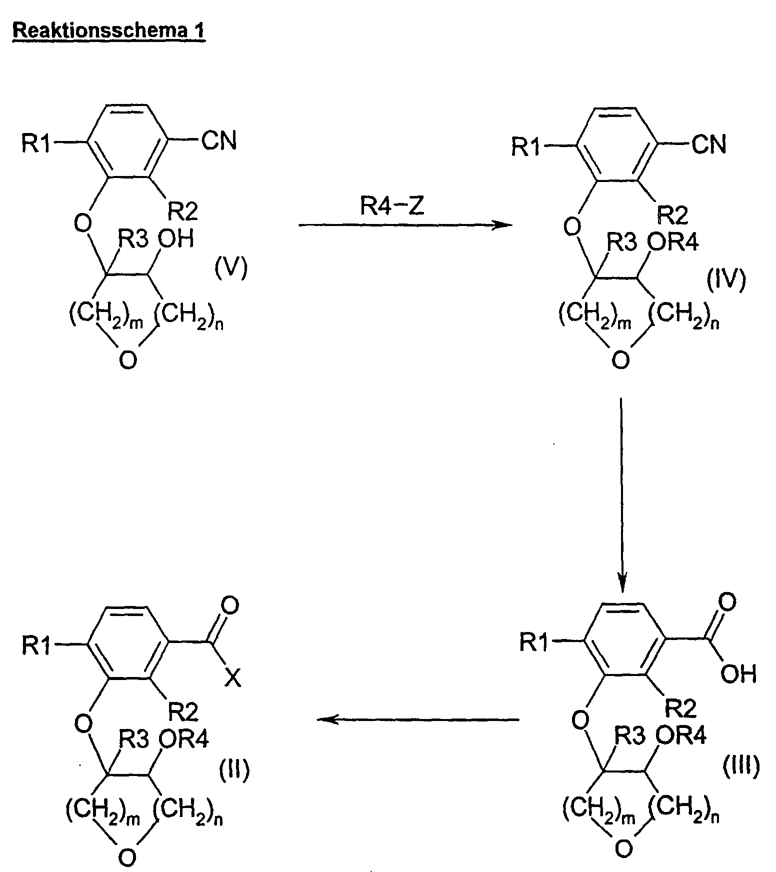

In einem ersten Reaktionsschritt werden die Verbindungen der Formel V, in denen R1, R2, R3, m und n die oben angegebenen Bedeutungen besitzen, einer Alkylierungsreaktion unterzogen.

Die Alkylierung wird in einem geeigneten inerten Lösungsmittel wie beispielsweise DMF, THF oder DMSO unter Zugabe einer Base, vorzugsweise Natriumhydrid, und einem Alkylierungsreagenz der Formel R4-Z, worin R4 die oben angegebenen Bedeutungen (Ausnahme R4≠H) hat und Z für eine geeignete Abgangsgruppe steht, durchgeführt.

Anschließend wird bei den erhaltenen Verbindungen der Formel IV die Cyanogruppe durch dem Fachmann bekannte Methoden zur Carboxylgruppe verseift (Verbindungen der Formel III). Die abschließende Aktivierung der Carboxylgruppe (beispielsweise durch Überführung in ein Säurehalogenid) liefert die Ausgangsverbindungen der Formel II.

Beispielhaft ist die Herstellung von Verbindungen der Formel II in den nachfolgenden Beispielen unter "Ausgangsverbindungen" beschrieben. Die Herstellung weiterer Verbindungen der Formel II kann auf analoge Weise erfolgen.

Die Amine R5-NH₂ sind bekannt, oder sie können auf bekannte Weise hergestellt werden.

Verbindungen der Formel V können nach dem allgemeinen Reaktionsschema 2 hergestellt werden:

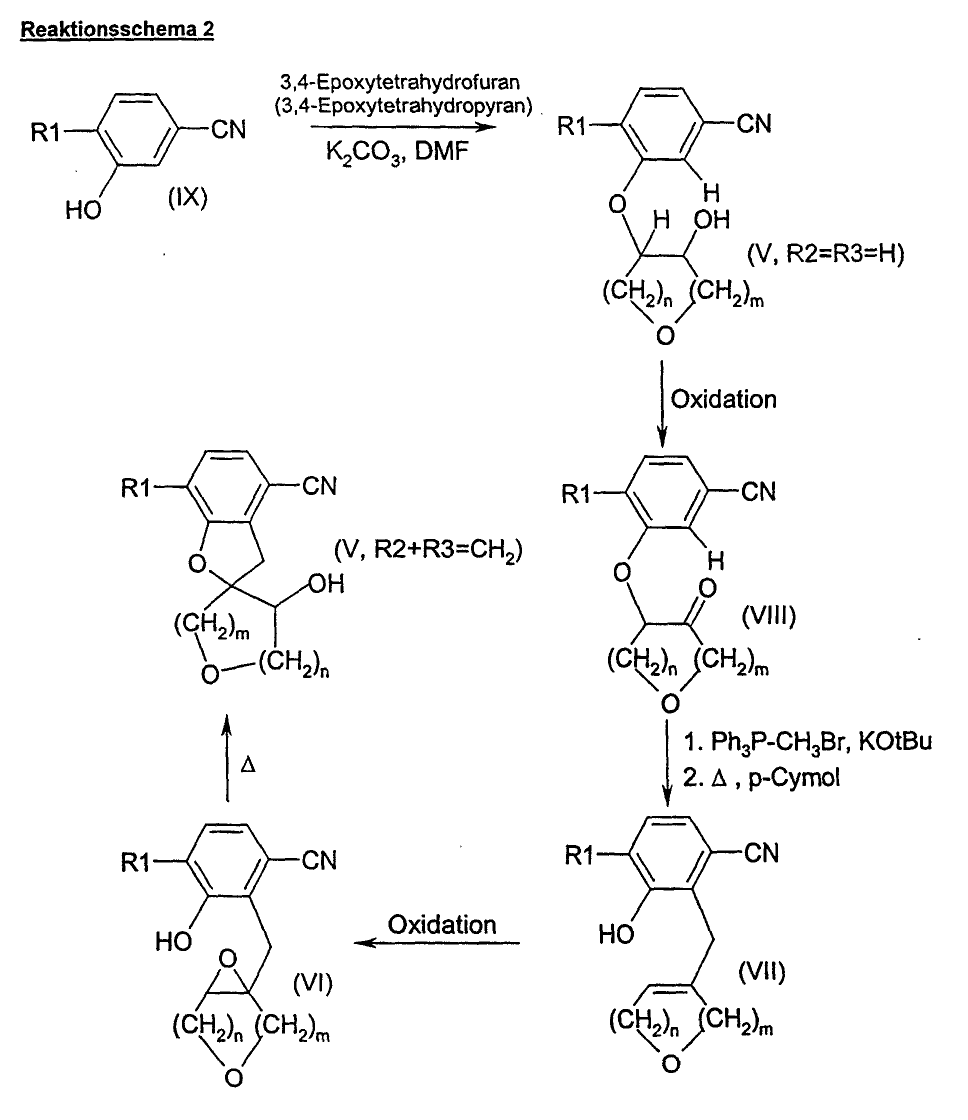

Exemplarisch ist die Herstellung von Verbindungen der Formel V (R2=R3=H bzw. R2+R3=CH₂), in denen R1, m und n die oben angegebenen Bedeutungen besitzen, in den nachfolgenden Beispielen unter "Ausgangsverbindungen" beschrieben. Die Herstellung weiterer Verbindungen der Formel V kann auf analoge Weise erfolgen.

In den nach dem Reaktionsschema 2 hergestellten Verbindungen der Formel V (R2=R3=H und R2+R3=CH₂) sind die Substituenten an den Chiralitätszentren vorzugsweise trans-konfiguriert.

Entsprechende Verbindungen der Formel V (R2=R3=H und R2+R3=CH₂), in denen die Substituenten an den Chiralitätszentren cis-konfiguriert sind, können durch den Fachmann bekannte Konfigurationsumkehrreaktionen hergestellt werden. Solche Reaktionen sind z.B. in Larock, Comprehensive Organic Transformations S. 479-480 (VCH 1989) beschrieben.

Das zweite Verfahren, daß sich bevorzugt zur Herstellung von Verbindungen der Formel I, in denen R1, R5, m und n die oben angegebenen Bedeutungen besitzen und R2, R3 und R4 für Wasserstoff stehen, ist dadurch gekennzeichnet, daß man Verbindungen der Formel X 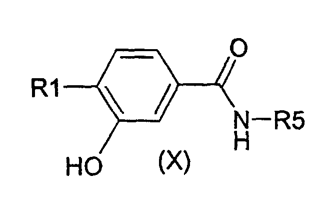 mit 3,4-Epoxytetrahydrofuran oder 3,4-Epoxytetrahydropyran umsetzt und daß man gewünschtenfalls anschließend erhaltene Verbindungen der Formel I in ihre Salze und/oder erhaltene Pyridine in die N-Oxide und gewünschtenfalls anschließend in die Salze überführt, oder daß man gewünschtenfalls anschließend erhaltene Salze der Verbindungen der Formel I in die freien Verbindungen überführt.

Die Umsetzung kann zum Beispiel in DMF als Lösungsmittel unter Zugabe von Kaliumcarbonat als Base, vorzugsweise bei erhöhter Temperatur, durchgeführt werden.

Verbindungen der Formel X können beispielsweise nach dem allgemeinen Reaktionsschema 3 mit oder ohne Einführung einer temporären Schutzgruppe (TP) an der phenolischen Hydroxygruppe hergestellt werden.

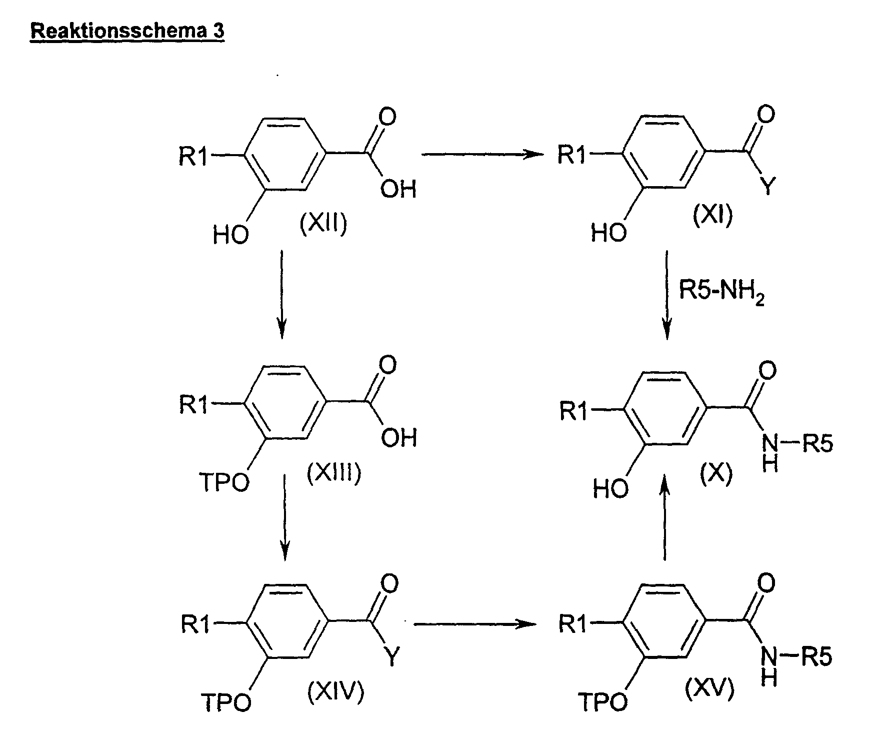

Hierbei wird zunächst bei den Verbindungen der Formel XII eine geeignete Abgangsgruppe Y (z.B. Y=CI oder Br) eingeführt. Die erhaltenen Verbindungen der Formel XI werden anschließend mit Aminen der Formel R5-NH₂ zu den Verbindungen der Formel X umgesetzt. Alternativ kann die Herstellung der Verbindungen der Formel X auch über die temporär geschützten Verbindungen der Formeln XIII, XIV und XV erfolgen.

Verbindungen der Formel XII sind bekannt, käuflich oder können nach bekannten Verfahren hergestellt werden.

Verbindungen der Formel I, in denen R1, R2, R3, R5, m und n die oben angegebenen Bedeutungen haben und R4 für Wasserstoff steht, lassen sich auch nach dem zuerst beschriebenen Verfahren herstellen, indem man anstelle des Substituenten R4 eine temporäre Schutzgruppe einführt und diese am Ende der Reaktionssequenz wieder abspaltet.

Dem Fachmann ist bekannt, daß es im Fall mehrerer reaktiver Zentren an einer Ausgangs- oder Zwischenverbindung notwendig sein kann, ein oder mehrere reaktive Zentren temporär durch Schutzgruppen zu blockieren, um eine Reaktion gezielt am gewünschten Reaktionszentrum ablaufen zu lassen.

Eine ausführliche Beschreibung zur Anwendung einer Vielzahl bewährter Schutzgruppen findet sich beispielsweise in T.W. Greene, Protective Groups in Organic Synthesis, John Wiley & Sons, 1991.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel (z. B. einem Keton, wie Aceton, Methylethylketon oder Methylisobutylketon, einem Ether, wie Diethylether, Tetrahydrofuran oder Dioxan, einem chlorierten Kohlenwasserstoff, wie Methylenchlorid oder Chloroform, oder einem niedermolekularen aliphatischen Alkohol wie Ethanol oder Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die nachfolgenden Beispiele dienen der näheren Erläuterung der Erfindung ohne sie einzuschränken. Ebenso können weitere Verbindungen der Formel I, deren Herstellung nicht explizit beschrieben ist, in analoger oder in einer dem Fachmann an sich vertrauten Weise unter Anwendung üblicher Verfahrenstechniken hergestellt werden.

In den Beispielen steht Schmp. für Schmelzpunkt, d für Tag(e), h für Stunde(n), min für Minute(n) und RT für Raumtemperatur. Die in den Beispielen genannten Verbindungen und ihre Salze sind bevorzugter Gegenstand der Erfindung.

### Endverbindungen:

### 1. N-(3,5-Dichlorpyridin-4-yl)-4-methoxy-3-(4-methoxy-tetrahydrofuran-3-yl-oxy)-benzamid

1,0 g (3,7 mmol) 4-Methoxy-3-(4-methoxy-tetrahydrofuran-3-yl-oxy)-benzoesäure (Ausgangsverbindung A1) wird in 4 ml Oxalylchlorid 2 h zum Rückfluß erhitzt. Man entfernt überschüssiges Oxalylchlorid im Vakuum und koevaporiert mit 2 x 20 ml Toluol. Zu einer Suspension von 220 mg (7,4 mmol) 80 %igem Natriumhydrid in 10 ml Tetrahydrofuran tropft man eine Lösung von 600 mg (3,7 mmol) 4-Amino-3,5-dichlorpyridin in 10 ml Tetrahydrofuran. Man rührt 1 h bei RT, tropft anschließend das Säurechlorid in weiteren 10 ml Tetrahydrofuran zu und läßt das Reaktionsgemisch über Nacht bei RT rühren. Man engt ein, versetzt den Rückstand mit 50 ml Wasser und extrahiert mit 3 x 50 ml Dichlormethan. Die vereinigten organischen Phasen werden über Magnesiumsulfat getrocknet, eingeengt und der Rückstand aus 50 ml Isopropanol kristallisiert. Man erhält 1,2 g der Titelverbindung vom Schmp. 175-177°C.

### 2. N-(3,5-Dichlorpyridin-4-yl)-3-(4-ethoxy-tetrahydrofuran-3-yl-oxy)-4-methoxybenzamid

1,0 g (3,5 mmol) 3-(4-Ethoxy-tetrahydrofuran-3-yl-oxy)-4-methoxy-benzoesäure (Ausgangsverbindung A2) werden analog Beispiel 1 umgesetzt und man erhält 1,0 g der Titelverbindung vom Schmp. 171-173°C.

### 3. N-(3,5-Dichlorpyridin-4-yl)-3-(4-benzyloxy-tetrahydrofuran-3-yl-oxy)-4-methoxybenzamid

1,0 g (2,9 mmol) 3-(4-Benzyloxy-tetrahydrofuran-3-yl-oxy)-4-methoxy-benzoesäure (Ausgangsverbindung A3) werden analog Beispiel 1 umgesetzt und man erhält 1,17 g der Titelverbindung vom Schmp. 190-191°C.

### 4. N-(3,5-Dichlorpyridin-4-yl)-2,3-dihydro-4',7-dimethoxybenzofuran-2-splro-3'-tetrahydrofuran-4-carbonsäureamid

700 mg (2,5 mmol) 2,3-Dihydro-4',7-dimethoxybenzofuran-2-spiro-3'-tetrahydrofuran-4-carbonsäure (Ausgangsverbindung A4) werden in 10 ml Oxalylchlorid 2 h zum Rückfluß erhitzt. Man entfernt überschüssiges Oxalylchlorid im Vakuum und koevaporiert mit 2 x 10 ml Toluol. Zu einer Suspension von 220 mg (5,5 mmol) 60 %igem Natriumhydrid in 5 ml Tetrahydrofuran tropft man eine Lösung von 900 mg (5,5 mmol) 4-Amino-3,5-dichlorpyridin in 10 ml Tetrahydrofuran. Man rührt 3 h bei RT, tropft anschließend das Säurechlorid in weiteren 15 ml Tetrahydrofuran zu und läßt das Reaktionsgemisch über Nacht bei RT rühren. Man engt ein, versetzt den Rückstand mit 50 ml Wasser und extrahiert mit 3 x 50 ml Dichlormethan. Die vereinigten organischen Phasen werden über Magnesiumsulfat getrocknet, eingeengt und der Rückstand über Kieselgel chromatographiert [Toluol/Ethylacetat/Essigsäure = 20:10:1]. Die produkthaltigen Fraktionen werden eingeengt und der Rückstand aus einem Wasser/Methanol-Gemisch kristallisiert. Man erhält 730 mg der Titelverbindung vom Schmp. 98°C [Sintern].

### 5. N-(3,5-Dichlorpyridin-4-yl)-2,3-dihydro-4'-benzyloxy-7-methoxybenzofuran-2-spiro-3'-tetrahydrofuran-4-carbonsäureamid

400 mg (1,12 mmol) 2,3-Dihydro-4'-benzyloxy-7-methoxybenzofuran-2-spiro-3'-tetrahydrofuran-4-carbonsäure (Ausgangsverbindung A5) werden in 3 ml Oxalylchlorid 90 min zum Rückfluß erhitzt. Man entfernt überschüssiges Oxalylchlorid im Vakuum und koevaporiert mit 2 x 10 ml Toluol. Zu einer Suspension von 100 mg (2,5 mmol) 60 %igem Natriumhydrid in 3 ml Tetrahydrofuran tropft man eine Lösung von 400 mg (2,5 mmol) 4-Amino-3,5-dichlorpyridin in 10 ml Tetrahydrofuran. Man rührt 90 min bei RT, tropft anschließend das Säurechlorid in weiteren 5 ml Tetrahydrofuran zu und läßt das Reaktionsgemisch 2 h bei RT rühren. Man engt ein, versetzt den Rückstand mit 50 ml Wasser und extrahiert mit 3 x 50 ml Dichlormethan. Die vereinigten organischen Phasen werden über Magnesiumsulfat getrocknet, eingeengt und der Rückstand über Kieselgel chromatographiert [Toluol/Ethylacetat = 4:1]. Die produkthaltigen Fraktionen werden eingeengt und der Rückstand aus Diisopropylether kristallisiert. Man erhält 270 mg der Titelverbindung vom Schmp. 82-84°C.

### 6. N-(3,5-Dichlorpyridin-4-yl)-3-(4-hydroxy-tetrahydrofuran-3-yl-oxy)-4-methoxy-benzamid

400 mg (1,2 mmol) N-(3,5-Dichlorpyridin-4-yl)-3-hydroxy-4-methoxy-benzamid (Ausgangsverbindung A16) und 540 mg (3,85 mmol) Kaliumcarbonat werden in 5 ml Dimethylformamid bei RT 1 h unter Stickstoffatmosphäre gerührt. Man erhitzt auf 120°C und gibt 4 x im Abstand von jeweils 6 h 115 mg (1,3 mmol) 3,4-Epoxy-tetrahydrofuran in das Reaktionsgemisch. Man engt ein, setzt 50 ml Wasser zu, säuert mit 2 N HCI an und extrahiert mit 3 x 50 ml Ethylacetat. Die vereinigten organischen Phasen werden mit gesättigter Natriumchlorid-Lösung gewaschen, über Magnesiumsulfat getrocknet, eingeengt und der Rückstand über Kieselgel chromatographiert [Toluol/Ethylacetat = 1:2]. Die produkthaltigen Fraktionen werden eingeengt und der Rückstand aus Diethylether kristallisiert. Man erhält 130 mg der Titelverbindung vom Schmp. 183-185°C.

### 7. N-(3,5-Dichlorpyridin-4-yl)-4-methoxy-3-(4-methylthiomethyloxy-tetrahydrofuran-3-yl-oxy)-benzamid

1,0 g (3,2 mmol) 4-Methoxy-3-(4-methylthiomethyloxy-tetrahydrofuran-3-yl-oxy)-benzoesäure (Ausgangsverbindung A19) werden in 4 ml Oxalylchlorid 90 min zum Rückfluß erhitzt. Man entfernt überschüssiges Oxalylchlorid im Vakuum und koevaporiert mit 2 x 20 ml Toluol. Zu einer Suspension von 200 mg (6,9 mmol) 80 %igem Natriumhydrid in 10 ml Tetrahydrofuran tropft man eine Lösung von 520 mg (3,2 mmol) 4-Amino-3,5-dichlorpyridin in 10 ml Tetrahydrofuran. Man rührt 90 min bei RT, tropft anschließend das Säurechlorid in weiteren 10 ml Tetrahydrofuran zu und läßt das Reaktionsgemisch 2 h bei RT rühren. Man engt ein, versetzt den Rückstand mit 25 ml Wasser und extrahiert mit 3 x 25 ml Ethylacetat. Die vereinigten organischen Phasen werden mit gesättigter Natriumchloridlösung gewaschen, über Magnesiumsulfat getrocknet, eingeengt und der Rücksand über Kieselgel chromatographiert. Die produkthaltigen Fraktionen werden eingeengt und der Rückstand aus Acetonitril kristallisiert. Man erhält 300 mg der Titelverbindung vom Schmp. 195-198°C.

### Ausgangsverbindungen:

### A1. 4-Methoxy-3-(4-methoxy-tetrahydrofuran-3-yl-oxy)-benzoesäure

2,0 g (8,0 mmol) 4-Methoxy-3-(4-methoxy-tetrahydrofuran-3-yl-oxy)-benzonitril (A6) und 4,2 g (80,0 mmol) Kaliumhydroxid werden in 20 ml Glycerin 3 h auf 140°C erhitzt. Man versetzt mit 50 ml Wasser und säuert durch Zutropfen von 40 ml 2 N Salzsäure unter Eiskühlung an. Man rührt 1 h nach, filtriert und wäscht den Niederschlag mit Eiswasser. Man erhält 1,79 g der Titelverbindung vom Schmp. 166-168°C.

### A2. 3-(4-Ethoxy-tetrahydrofuran-3-yl-oxy)-4-methoxy-benzoesäure

1,46 g (5,5 mmol) 3-(4-Ethoxy-tetrahydrofuran-3-yl-oxy)-4-methoxy-benzonitril (A7) und 3,0 g (55,0 mmol) Kaliumhydroxid werden in 15 ml Glycerin 3 h auf 140°C erhitzt. Man versetzt mit 50 ml Wasser, wäscht mit 2 x 25 ml Ethylacetat und säuert die wäßrige Phase durch Zutropfen von 30 ml 2 N Salzsäure unter Eiskühlung an. Man rührt 1 h nach, filtriert und wäscht den Niederschlag mit Eiswasser. Man erhält 1,49 g der Titelverbindung vom Schmp. 135-138°C.

### A3. 3-(4-Benzyloxy-tetrahydrofuran-3-yl-oxy)-methoxy-benzoesäure

2,8 g (8,6 mmol) 3-(4-Benzyloxy-tetrahydrofuran-3-yl-oxy)-4-methoxy-benzonitril (A8) und 4,8 g (86,0 mmol) Kaliumhydroxid werden in 30 ml Glycerin 3 h auf 140°C erhitzt. Man versetzt mit 70 ml Wasser und säuert durch Zutropfen von 40 ml 2 N Salzsäure unter Eiskühlung an. Man rührt 1 h nach, filtriert und wäscht den Niederschlag mit Eiswasser. Zur weiteren Reinigung wird das Produkt über Kieselgel chromatographiert [Toluol/Ethylacetat = 2:1]. Die produkthaltigen Fraktionen werden eingeengt und der Rückstand aus Isopropanol kristallisiert. Man erhält 2,1 g der Titelverbindung vom Schmp. 153-156°C.

### A4. 2,3-Dihydro-4',7-dimethoxybenzofuran-2-spiro-3'-tetrahydrofuran-4-carbonsäure

1,95 g (7,5 mmol) 2,3-Dihydro-4-cyano-4',7-dimethoxybenzofuran-2-spiro-3'-tetrahydrofuran (A9) und 4,2 g (75,0 mmol) Kaliumhydroxid werden in 40 ml Glycerin 3 h auf 140°C erhitzt. Man versetzt mit 150 ml Wasser und säuert durch Zutropfen von 30 ml 2 N Salzsäure unter Eiskühlung an. Man rührt 1 h nach, filtriert und wäscht den Niederschlag mit Eiswasser. Man erhält 1,97 g der Titelverbindung vom Schmp. 231-232°C.

### A5. 2,3-Dihydro-4'-benzyloxy-7-methoxybenzofuran-2-spiro-3'-tetrahydrofuran-4-carbonsäure

650 mg (1,92 mmol) 2,3-Dihydro-4'-benzyloxy-4-cyano-7-methoxybenzofuran-2-spiro-3'-tetrahydrofuran (A10) und 1,35 g (19 mmol) Kaliumhydroxid werden in 15 ml Glycerin 5 h auf 150°C erhitzt. Man versetzt mit 40 ml Wasser und säuert durch Zutropfen von 10 ml 2 N Salzsäure unter Eiskühlung an. Man rührt 1 h nach, filtriert und wäscht den Niederschlag mit Eiswasser. Man erhält 610 mg der Titelverbindung vom Schmp. 196-198°C.

### A6. 4-Methoxy-3-(4-methoxy-tetrahydrofuran-3-yl-oxy)-benzonitril

Zu einer Lösung von 140 mg (4,6 mmol) 80 %igem Natriumhydrid in 5 ml Dimethylformamid tropft man unter Stickstoffatmosphäre 1,0 g (4,2 mmol) 3-(4-Hydroxy-tetrahydrofuran-3-yl-oxy)-4-methoxybenzonitril (A15) in 5 ml Dimethylformamid und erhitzt 20 min auf 60°C. Anschließend werden bei RT 315 µl (5,0 mmol) Methyljodid, gelöst in 1 ml Dimethylformamid, zugetropft. Man rührt 2 h bei RT, engt ein, versetzt mit 20 ml Wasser und säuert mit 2 N Salzsäure an. Nach Extraktion mit 3 x 30 ml Ethylacetat werden die vereinigten organischen Phasen über Magnesiumsulfat getrocknet, eingeengt und das Rohprodukt aus 10 ml Isopropanol umkristallisiert. Man erhält 760 mg der Titelverbindung vom Schmp. 98-100°C.

### A7. 3-(4-Ethoxy-tetrahydrofuran-3-yl-oxy)-4-methoxy-benzonitril

Zu einer Lösung von 140 mg (4,6 mmol) Natriumhydrid in 5 ml Dimethylformamid tropft man unter Stickstoffatmosphäre 1,0 g (4,2 mmol) 3-(4-Hydroxy-tetrahydrofuran-3-yl-oxy)-4-methoxy-benzonitril (A15) in 5 ml Dimethylformamid und erhitzt 1 h auf 40°C. Anschließend werden bei RT 600 µl (7,5 mmol) Ethyljodid, gelöst in 1 ml Dimethylformamid, zugetropft. Man rührt 2 h bei RT, engt ein, versetzt mit 25 ml Wasser und säuert mit 2 N Salzsäure an. Nach Extraktion mit 3 x 25 ml Ethylacetat werden die vereinigten organischen Phasen mit gesättigter Natriumchloridlösung gewaschen, über Magnesiumsulfat getrocknet und eingeengt. Zur weiteren Reinigung wird das Produkt über Kieselgel chromatographiert [Toluol/Ethylacetat = 4:1]. Die produkthaltigen Fraktionen werden eingeengt und der Rückstand aus Isopropanol kristallisiert. Man erhält 660 mg der Titelverbindung vom Schmp. 64-66°C.

### A8. 3-(4-Benzyloxy-tetrahydrofuran-3-yl-oxy)-4-methoxy-benzonitril

Zu einer Lösung von 350 mg (12,0 mmol) Natriumhydrid in 12 ml Dimethylformamid tropft man unter Stickstoffatmosphäre 2,5 g (11,0 mmol) 3-(4-Hydroxy-tetrahydrofuran-3-yl-oxy)-4-methoxy-benzonitril (A 15) in 12 ml Dimethylformamid und erhitzt 1 h auf 40°C. Anschließend werden bei RT 1,6 ml (13,0 mmol) Benzylchlorid, gelöst in 6 ml Dimethylformamid, zugetropft. Man rührt 4 h bei RT, engt ein, versetzt mit 50 ml Wasser und säuert mit 2 N Salzsäure an. Nach Extraktion mit 3 x 40 ml Ethylacetat werden die vereinigten organischen Phasen mit gesättigter Natriumchloridlösung gewaschen, über Magnesiumsulfat getrocknet und eingeengt. Zur weiteren Reinigung wird das Produkt über Kieselgel chromatographiert [Toluol/Ethylacetat = 4:1]. Die produkthaltigen Fraktionen werden eingeengt und der Rückstand aus Isopropanol kristallisiert. Man erhält 3,05 g der Titelverbindung vom Schmp. 72-75°C.

### A9. 2,3-Dihydro-4-cyano-4',7-dimethoxybenzofuran-2-spiro-3'-tetrahydrofuran

Zu einer Lösung von 270 mg (8,8 mmol) 80 %igem Natriumhydrid in 10 ml Dimethylformamid tropft man unter Stickstoffatmosphäre 2,0 g (8,0 mmol) 2,3-Dihydro-4-cyano-4'-hydroxy-7-methoxybenzofuran-2-spiro-3'-tetrahydrofuran (A11) in 15 ml Dimethylformamid und erhitzt 1 h auf 40°C. Anschließend werden bei RT 700 µl (10,6 mmol) Methyljodid, gelöst in 5 ml Dimethylformamid, zugetropft. Man rührt 2 h bei RT, engt ein, und kristallisiert aus 50 ml Wasser. Man erhält 1,97 g der Titelverbindung vom Schmp. 151-156°C.

### A10. 2,3-Dihydro-4'-benzyloxy-4-cyano-7-methoxybenzofuran-2-spiro-3'-tetrahydrofuran

Zu einer Lösung von 110 mg (3,65 mmol) 80 %igem Natriumhydrid in 2 ml Dimethylformamid tropft man unter Stickstoffatmosphäre 750 mg (3,03 mmol) 2,3-Dihydro-4-cyano-4'-hydroxy-7-methoxybenzofuran-2-spiro-3'-tetrahydrofuran (A11) in 10 ml Dimethylformamid und erhitzt 90 min auf 40°C. Anschließend werden bei RT 470 µl (10.6 mmol) Benzylbromid, gelöst in 10 ml Dimethylformamid, zugetropft. Man rührt 4 h bei RT, engt ein und versetzt mit 25 ml Wasser. Nach Extraktion mit 3 x 25 ml Ethylacetat werden die vereinigten organischen Phasen über Magnesiumsulfat getrocknet und eingeengt. Zur weiteren Reinigung wird das Produkt über Kieselgel chromatographiert [Toluol/Ethylacetat = 9:1]. Die produkthaltigen Fraktionen werden eingeengt und der Rückstand aus Diisopropylester kristallisiert. Man erhält 740 mg der Titelverbindung vom Schmp. 146-147°C.

### A11. 2,3-Dihydro-4-cyano-4'-hydroxy-7-methoxybenzofuran-2-spiro-3'-tetrahydrofuran

2,3 g (9,5 mmol) 2-(3,4-Epoxy-tetrahydrofuran-3-yl-methyl)-3-hydroxy-4-methoxy-benzonitril (A12) werden 3 h auf 140°C erhitzt. Man kristallisiert aus heißem Methanol und erhält 1,7 g der Titelverbindung vom Schmp. 171-176°C.

### A12. 2-(3,4-Epoxy-tetrahydrofuran-3-yl-methyl)-3-hydroxy-4-methoxy-benzonitril

2,3 g (10,0 mmol) 2-(2,5-Dihydrofuran-3-yl-methyl)-3-hydroxy-4-methoxy-benzonitril (A13) und 2,7 g (11 mmol) 70-75 %ige m-Chlorperbenzoesäure werden in 50 ml Dichlormethan über Nacht bei RT gerührt. Man wäscht das Reaktionsgemisch nacheinander mit gesättigter Natriumhydrogencarbonat-, Natriumdisulfit- und Natriumchlorid-Lösung, trocknet über Magnesiumsulfat und engt die organische Phase ein. Das Rohprodukt wird ohne weitere Reinigung umgesetzt.

### A13. 2-(2,5-Dihydrofuran-3-yl-methyl)-3-hydroxy-4-methoxy-benzonitril

46,4 g (128,0 mmol) Methyltriphenylphosphoniumbromid und 14,6 g (128,0 mmol) Kalium-t-butylat werden in 300 ml Tetrahydrofuran 3 h bei RT unter Stickstoffatmosphäre gerührt. Man kühlt auf 0°C, gibt 25,3 g (108,0 mmol) 3-(Tetrahydrofuran-4-on-3-yl-oxy)-4-methoxy-benzonitril (A14) in 200 ml Tetrahydrofuran zu und rührt 4 h unter Eiskühlung. Anschließend wird weitgehend eingeengt und mit 300 ml Wasser hydrolysiert. Man extrahiert mit insgesamt 900 ml Ethylacetat, trocknet die vereinigten organischen Phasen über Magnesiumsulfat und engt ein. Das Rohprodukt wird über Kieselgel filtriert [To-Iuol/Ethylacetat = 4:1] und anschließend in 400 ml p-Cymol 8 h auf 150°C erhitzt. Das Produkt kristallisiert beim Abkühlen aus der Reaktionslösung und man erhält 17,75 g der Titelverbindung vom Schmp. 142-144°C.

### A14. 3-(Tetrahydrofuran-4-on-3-yl-oxy)-4-methoxy-benzonitril

Zu einer Lösung von 1,27 (12,75 mmol) Chrom-(VI)-oxid in 50 ml Dichlormethan werden bei 0°C 2,05 ml (25,5 mmol) Pyridin und 1,2 ml (12,75 mmol) Acetanhydrid zugetropft. Nach 15 min bei RT wird 1,0 g (4,25 mmol) 3-(4-Hydroxy-tetrahydrofuran-3-yl-oxy)-4-methoxy-benzonitril (A15) in 25 ml Dichlormethan zugetropft. Man läßt 1 h bei RT rühren und filtriert das Reaktionsgemisch anschließend über Celite. Das Filtrat wird mit Wasser versetzt und mit 2 N Salzsäure angesäuert. Man trennt die Phasen, extrahiert die wäßrige Phase mit Dichlormethan, trocknet die vereinigten organischen Phasen über Magnesiumsulfat und engt ein. Zur weiteren Reinigung wird das Produkt über Kieselgel chromatographiert [Ethylacetat]. Die produkthaltigen Fraktionen werden eingeengt und der Rückstand aus Diisopropylester kristallisiert. Man erhält 730 mg der Titelverbindung vom Schmp. 147-149°C.

### A15. 3-(4-Hydroxy-tetrahydrofuran-3-yl-oxy)-4-methoxy-benzonitril

50 g (335 mmol) 3-Hydroxy-4-methoxy-benzonitril und 92 g (670 mmol) Kaliumcarbonat werden in 900 ml Dimethylformamid aufgeschlämmt und 1 h bei RT gerührt. Anschließend tropft man 32 g (368 mmol) 3,4-Epoxy-tetrahydrofuran zu und rührt 6 h bei 90°C. Das Lösungsmittel wird im Vakuum abgedampft und der Rückstand zwischen 500 ml Ethylacetat und 500 ml Wasser verteilt. Man trennt die Phasen, extrahiert die wäßrige Phase mit 4 x 250 ml Ethylacetat, wäscht die vereinigten organischen Phasen mit gesättigter Natriumchlorid-Lösung, trocknet über Magnesiumsulfat und engt ein. Das Rohprodukt wird in 400 ml Toluol umkristallisiert. Man erhält 62 g der Titelverbindung vom Schmp. 147-149°C.

### A16. N-(3,5-Dichlorpyridin-4-yl)-3-hydroxy-4-methoxy-benzamid

Eine Suspension von 1,0 g (2,5 mmol) N-(3,5-Dichlorpyridin-4-yl)-4-methoxy-3-pivaloyloxy-benzamid (A17) in 5 ml Methanol wird mit 325 mg (6,0 mmol) Natriummethanolat in 5 ml Methanol versetzt und 4 h auf 50°C erhitzt. Man läßt abkühlen, engt ein, versetzt mit 50 ml Wasser, säuert mit 2 N Salzsäure an und extrahiert mit 4 x 50 ml Ethylacetat. Die vereinigten organischen Phasen werden über Magnesiumsulfat getrocknet, eingeengt und das Rohprodukt aus 20 ml Isopropanol kristallisiert. Man erhält 870 mg der Titelverbindung vom Schmp. 231-233°C.

### A17. N-(3,5-Dichlorpyridin-4-yl)-4-methoxy-3-pivaloyloxy-benzamid

1,0 g (4,0 mmol) 4-Methoxy-3-pivaloyloxy-benzoesäure (A18) werden in 10 ml Oxalylchlorid 2 h zum Rückfluß erhitzt. Man entfernt überschüssiges Oxalylchlorid im Vakuum und koevaporiert mit 2 x 20 ml Toluol. Zu einer Suspension von 240 mg (8,0 mmol) 80 %igem Natriumhydrid in 10 ml Tetrahydro-furan tropft man eine Lösung von 650 mg (4,0 mmol) 4-Amino-3,5-dichlorpyridin in 10 ml Tetrahydrofuran. Man rührt 1 h bei RT, tropft anschließend das Säurechlorid in weiteren 10 ml Tetrahydrofuran zu und läßt das Reaktionsgemisch 3 h bei RT rühren. Man engt ein, versetzt den Rückstand mit 20 ml Wasser und 20 ml gesättigter Natriumchlorid-Lösung und extrahiert mit 3 x 50 ml Ethylacetat. Die vereinigten organischen Phasen werden über Magnesiumsulfat getrocknet, eingeengt und das Rohprodukt aus 15 ml Diisopropylether umkristallisiert. Man erhält 1,29 g der Titelverbindung vom Schmp. 177-179°C.

### A18. 4-Methoxy-3-pivaloyloxy-benzoesäure

2,0 g (12,0 mmol) Isovanillinsäure werden in 15 ml Pyridin und 15 ml Toluol gelöst und mit 3,8 ml (32,0 mmol) Pivaloylchlorid versetzt. Man rührt 48 h bei RT. Anschließend wird das Reaktionsgemisch mit 20 ml 2 N Natronlauge versetzt und 1 h zum Rückfluß erhitzt. Man läßt abkühlen, säuert mit halbkonzentrierter Salzsäure auf pH = 1 an und extrahiert mit 2 x 50 ml Toluol. Die vereinigten organischen Phasen werden über Magnesiumsulfat getrocknet, eingeengt und das Produkt aus 25 ml Toluol kristallisiert. Man erhält 1,1 g der Titelverbindung vom Schmp. 182-183°C.

### A19. 4-Methoxy-3-(4-methylthiomethyloxy-tetrahydrofuran-3-yl-oxy)-benzoesäure

6,5 g (22,0 mmol) 4-Methoxy-3-(4-methylthiomethyloxy-tetrahydrofuran-3-yl-oxy)-benzonitril (A20) und 12,3 g (220 mmol) Kaliumhydroxid werden in 60 ml Glycerin 3 h auf 130°C erhitzt. Anschließend wird mit 100 ml Wasser versetzt und durch Zutropfen 2 N Salzsäure auf pH=5 angesäuert. Man extrahiert mit insgesamt 900 ml Ethylacetat, trocknet die vereinigten organischen Phasen über Magnesiumsulfat, engt ein und rührt den Rückstand in heißem Ethylacetat aus. Nach Umkristallisation aus Isopropanol erhält man 5,15 g der Titelverbindung vom Schmp. 144-145°C.

### A20. 4-Methoxy-3-(4-methylthiomethyloxy-tetrahydrofuran-3-yl-oxy)-benzonitril

31,0 g (132 mmol) 3-(4-Hydroxy-tetrahydrofuran-3-yl-oxy)-4-methoxy-benzonitril (A15) werden in 500 ml Acetanhydrid und 180 ml Dimethylsulfoxid 2 d bei RT gerührt. Man engt ein und kristallisiert den Rückstand aus 300 ml Isopropanol um. Das Rohprodukt wird nochmals aus 500 ml Ethanol umkristallisiert. Man erhält 6,6 g der Titelverbindung vom Schmp. 124-125°C.

Die erfindungsgemäßen Verbindungen besitzen wertvolle pharmakologische Eigenschaften, die sie gewerblich verwertbar machen. Als selektive Zyklisch-Nukleotid Phosphodiesterase (PDE) Inhibitoren (und zwar des Typs 4) eignen sie sich einerseits als Bronchialtherapeutika (zur Behandlung von Atemwegsobstruktionen aufgrund ihrer dilatierenden aber auch aufgrund ihrer atemfrequenz- bzw. atemantriebssteigernden Wirkung) und zur Behebung von erektiler Dysfunktion aufgrund der gefäßdilatierenden Wirkung, andererseits jedoch vor allem zur Behandlung von Erkrankungen, insbesondere entzündlicher Natur, z.B. der Atemwege (Asthma-Prophylaxe), der Haut, des zentralen Nervensystems, des Darms, der Augen und der Gelenke, die vermittelt werden durch Mediatoren, wie Histamin, PAF (Plättchen-aktivierender Faktor), Arachidonsäure-Abkömmlinge wie Leukotriene und Prostaglandine, Zytokine, Interleukine, Chemokine, alpha-, beta- und gamma-Interferon, Tumornekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale, Emphysema, COPD); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS, Multiple Sklerose), Graft versus Host Reaktionen, Transplantationsabstoßungsreaktionen, Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise erektile Dysfunktion oder Koliken der Nieren und der Harnleiter im Zusammenhang mit Nierensteinen. Desweiteren können die erfindungsgemäßen Verbindungen zur Behandlung von Diabetes Insipidus und Erkrankungen im Zusammenhang mit Störungen des Hirnstoffwechsels, wie z. B. zerebrale Senilität, Senile Demenz (Alzheimer Demenz), Multiinfarkt Demenz oder auch Erkrankungen des ZNS, wie beispielsweise Depressionen oder arteriosklerotische Demenz eingesetzt werden.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe von Krankheiten, insbesondere den genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Ein weiterer Gegenstand der Erfindung ist ein Handelsprodukt, bestehend aus einem üblichen Sekundärpackmittel, einem das Arzneimittel enthaltenden Primärpackmittel (beispielsweise eine Ampulle oder ein Blister) und gewünschtenfalls einen Beipackzettel, wobei das Arzneimittel antagonistische Wirkung gegen zyklisch-nukleotid Phosphodiesterasen des Typs 4 (PDE4) zeigt und zur Abschwächung der Symptome von Krankheiten führt, die in Zusammenhang mit zyklisch-nukleotid Phosphodiesterasen des Typs 4 stehen, und wobei auf dem Sekundärpackmittel oder auf dem Beipackzettel des Handelsprodukts auf die Eignung des Arzneimittels zur Prophylaxe oder Behandlung von Krankheiten, die im Zusammenhang mit zyklisch-nukleotid Phosphodiesterasen des Typs 4 stehen, hingewiesen wird, und wobei das Arzneimittel ein oder mehrere erfindungsgemäße Verbindungen der Formel I enthält. Das Sekundärpackmittel, das das Arzneimittel enthaltende Primärpackmittel und der Beipackzettel entsprechen ansonsten dem, was der Fachmann als Standard für Arzneimittel dieser Art ansehen würde.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,1 und 3 mg pro Tag. Die übliche Dosis bei systemischer Therapie (p. o. oder i. v.) liegt zwischen 0,03 und 3 mg pro Kilogramm und Tag.

Bei der Untersuchung der PDE 4-Hemmung auf zellulärer Ebene kommt der Aktivierung von Entzündungszellen besondere Bedeutung zu. Als Beispiel sei die FMLP (N-formyl-methionyl-leucyl-phenylalanin)-induzierte Superoxid-Produktion von neutrophilen Granulozyten genannt, die als Luminolverstärkte Chemilumineszenz gemessen werden kann. [Mc Phail LC, Strum SL, Leone PA und Sozzani S, The neutrophil respiratory burst mechanism. In "Immunology Series" **1992,** 57, 47-76; ed. Coffey RG (Marcel Decker, Inc., New York-Basel-Hong Kong)].

Substanzen, welche die Chemilumineszenz sowie die Zytokinsekretion und die Sekretion entzündungssteigernder Mediatoren an Entzündungszellen, insbesonders neutrophilen und eosinophilen Granulozyten, T-Lymphozyten, Monozyten und Makrophagen hemmen, sind solche, welche die PDE 4 hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE 4-Hemmung durch die erfindungsgemäßen Substanzen ist damit ein zentraler Indikator für die Unterdrückung von entzündlichen Prozessen. (**Giembycz MA,** Could isoenzyme-selective phosphodiesterase inhibitors render bronchodilatory therapy redundant in the treatment of bronchial asthma?. Biochem Pharmacol **1992,** 43, 2041-2051; **Torphy TJ et al.,** Phosphodiesterase inhibitors: new opportunities for treatment of asthma. Thorax **1991,** 46, 512-523; **Schudt C** et al., Zardaverine: a cyclic AMP PDE 3/4 inhibitor. In "New Drugs for Asthma Therapy", 379-402, Birkhäuser Verlag Basel 1991; **Schudt C** et al., Influence of selective phosphodiesterase inhibitors on human neutrophil functions and levels of cAMP and Ca; Naunyn-Schmiedebergs Arch Pharmacol **1991,** 344, 682-690; **Tenor H** und **Schudt C,** Analysis of PDE isoenzyme profiles in cells and tissues by pharmacological methods. In "Phosphodiesterase Inhibitors", 21-40, "The Handbook of Immunopharmacology", Academic Press, 1996; **Hatzelmann A** et al., Enzymatic and functional aspects of dualselective PDE3/4-inhibitors. In "Phosphodiesterase Inhibitors", 147-160, "The Handbook of Immunopharmacology", Academic Press, 1996.

### Hemmung der PDE4-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. **1980,** 311, 193-198). Hierbei erfolgt im ersten Schritt die PDE-Reaktion. In einem zweiten Schritt wird das entstandene 5'-Nukleotid durch eine 5'-Nukieotidase des Schlangengiftes von Crotalus Atrox zum ungeladenen Nukleosid gespalten. Im dritten Schritt wird das Nukleosid auf lonenaustauschsäulen vom verbliebenen geladenen Substrat getrennt. Die Säulen werden mit 2 ml 30 mM Ammoniumformiat (pH 6,0) direkt in Minivials eluiert, in die noch 2 ml Szintillatorflüssigkeit zur Zählung gegeben wird.

Die für die erfindungsgemäßen Verbindungen ermittelten Hemmwerte [Hemmkonzentration als -log IC₅₀ (mol/l)] ergeben sich aus der folgenden Tabelle A, in der die Nummern der Verbindungen den Nummern der Beispiele entsprechen.

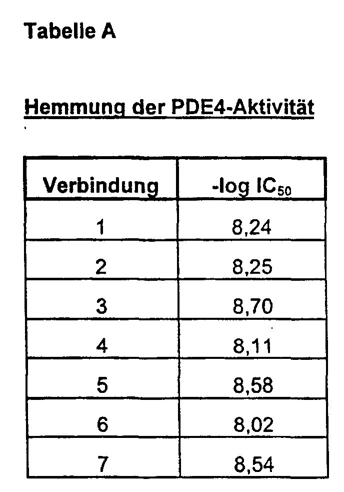

## Patentansprüche

1. Verbindungen der Formel I 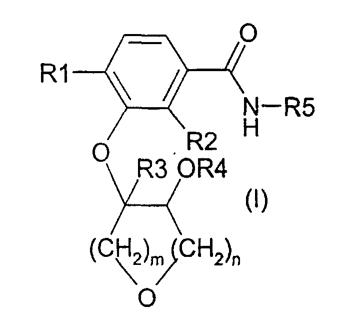 worin
R1 1-6C-Alkoxy, 3-7C-Cycloalkoxy, 3-7C-Cycloalkylmethoxy, Benzyloxy oder ganz oder überwiegend durch Fluor substituiertes 1-4C-Alkoxy bedeutet,
R2 Wasserstoff und
R3 Wasserstoff bedeutet
oder
R2 und R3 gemeinsam eine Methylengruppe darstellen,
R4 Wasserstoff, 1-8C-Alkyl, 1-6C-Alkoxy-1-4C-alkyl, 1-6C-Alkylthio-1-4-alkyl, 1-6C-Alkylsulfinyl-1-4C-alkyl, 1-6C-Alkylsulfonyl-1-4C-alkyl, 1-8C-Alkylcarbonyl, 3-7C-Cycloalkyl, 3-7C-Cycloalkylmethyl, Phenyl-1-4C-alkyl oder ganz oder überwiegend durch Fluor substituiertes 1-4C-Alkyl bedeutet,
R5 Phenyl, Pyridyl, durch R51, R52 und R53 substituiertes Phenyl oder durch R54, R55, R56 und R57 substituiertes Pyridyl bedeutet, wobei
R51 Hydroxy, Halogen, Cyano, Carboxyl, Trifluormethyl, 1-4C-Alkyl, 1-4C-Alkoxy, 1-4C-Alkoxycarbonyl, 1-4C-Alkylcarbonyl, 1-4C-Alkylcarbonyloxy, Amino, Mono- oder Di-1-4C-alkylamino oder 1-4C-Alkylcarbonylamino,
R52 Wasserstoff, Hydroxy, Halogen, Amino, Trifluormethyl, 1-4C-Alkyl oder 1-4C-Alkoxy,
R53 Wasserstoff, Halogen, 1-4C-Alkyl oder 1-4C-Alkoxy,
R54 Hydroxy, Halogen, Cyano, Carboxyl, 1-4C-Alkyl, 1-4C-Alkoxy, 1-4C-Alkoxycarbonyl oder Amino,
R55 Wasserstoff, Halogen, Amino oder 1-4C-Alkyl,
R56 Wasserstoff oder Halogen und
R57 Wasserstoff oder Halogen bedeutet,
n für 1 oder 2 steht,
m für 1 oder 2 steht,
wobei die Summe aus m und n nur die Werte 2 oder 3 annehmen darf,
die Salze dieser Verbindungen sowie die N-Oxide der Pyridine und deren Salze.

2. Verbindungen der Formel I nach Anspruch 1, in denen
R1 1-4C-Alkoxy, 3-5C-Cycloalkoxy, 3-5C-Cycloalkylmethoxy oder ganz oder überwiegend durch Fluor substituiertes 1-4C-Alkoxy bedeutet,
R2 Wasserstoff und
R3 Wasserstoff bedeutet,
oder
R2 und R3 gemeinsam eine Methylengruppe darstellen,
R4 Wasserstoff, 1-6C-Alkyl, 1-4C-Alkoxy-1-4C-alkyl, 1-4C-Alkylthio-1-4C-alkyl, 1-6C-Alkylcarbonyl, 3-7C-Cycloalkyl, 3-7C-Cycloalkylmethyl, Phenyl-1-4C-alkyl oder ganz oder überwiegend durch Fluor substituiertes 1-4C-Alkyl bedeutet,
R5 Phenyl, Pyridyl, durch R51, R52 und R53 substituiertes Phenyl oder durch R54, R55, R56 und R57 substituiertes Pyridyl bedeutet, wobei
R51 Halogen, Cyano, Carboxyl, 1-4C-Alkyl, 1-4C-Alkoxy oder 1-4C-Alkoxycarbonyl,
R52 Wasserstoff, Halogen, 1-4C-Alkyl oder 1-4C-Alkoxy,
R53 Wasserstoff, Halogen, 1-4C-Alkyl oder 1-4C-Alkoxy,
R54 Halogen oder 1-4C-Alkyl,
R55 Wasserstoff oder Halogen,
R56 Wasserstoff oder Halogen und
R57 Wasserstoff oder Halogen bedeutet,
n für 1 oder 2 steht,
m für 1 oder 2 steht,
wobei die Summe aus m und n nur die Werte 2 oder 3 annehmen darf,
die Salze dieser Verbindungen sowie die N-Oxide der Pyridine und deren Salze.

3. Verbindungen der Formel I nach Anspruch 1, in denen
R1 Methoxy oder Difluormethoxy bedeutet,
R2 Wasserstoff und
R3 Wasserstoff bedeutet
oder
R2 und R3 gemeinsam eine Methylengruppe darstellen,
R4 Wasserstoff, 1-6C-Alkyl, 1-4C-Alkoxy-1-4C-alkyl, 1-4C-Alkylthio-1-4C-alkyl, 1-4C-Alkylcarbonyl, Phenethyl oder Benzyl bedeutet,
R5 2-Bromphenyl, 2-Chlorphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2-Chlor-6-fluorphenyl, 2,6-Difluorphenyl, 2,6-Dichlorphenyl, 3,5-Dichlorpyrid-4-yl, 3-Methylpyrid-2-yl, 2-Chlorpyrid-3-yl, 3-Chlorpyrid-4-yl, 3,5-Dibrompyrid-2-yl, 3,5-Difluorpyrid-4-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
n für 1 steht,
m für 1 steht und
die Salze dieser Verbindungen sowie die N-Oxide der Pyridine und deren Salze.

4. Verbindungen der Formel I nach Anspruch 1, in denen
R1 Methoxy oder Difluormethoxy bedeutet,
R2 Wasserstoff und
R3 Wasserstoff bedeutet,
oder
R2 und R3 gemeinsam eine Methylengruppe darstellen,
R4 Wasserstoff, 1-4C-Alkyl, 1-2C-Alkoxy-1-2C-alkyl, 1-2C-Alkylthio-1-2C-alkyl, 1-4C-Alkylcarbonyl oder Benzyl bedeutet,
R5 3,5-Dichlorpyrid-4-yl, 2-Chlorpyrid-3-yl, 3-Chlorpyrid4-yl, 3,5-Dibrompyrid-2-yl, 3,5-Difluorpyrid-4-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
n für 1 steht,
m für 1 steht und
die Salze dieser Verbindungen sowie die N-Oxide der Pyridine und deren Salze.

5. Verbindungen der Formel I nach Anspruch 1, in denen
R1 Methoxy bedeutet,
R2 Wasserstoff und
R3 Wasserstoff bedeutet,
oder
R2 und R3 gemeinsam eine Methylengruppe darstellen,
R4 Wasserstoff, Methyl, Ethyl, Methylthiomethyl oder Benzyl bedeutet,
R5 3,5-Dichlorpyrid-4-yl bedeutet,
n für 1 steht,
m für 1 steht und
die Salze dieser Verbindungen.

6. Verbindungen nach Anspruch 1, 2, 3, 4 oder 5, in denen R2 und R3 jeweils Wasserstoff bedeuten.

7. Verbindungen nach Anspruch 1, 2, 3, 4 oder 5, in denen R2 und R3 gemeinsam eine Methylengruppe darstellen.

8. Arzneimittel enthaltend eine oder mehrere Verbindungen nach Anspruch 1 zusammen mit üblichen pharmazeutischen Hilfs- und/oder Trägerstoffen.

9. Verbindungen nach Anspruch 1 zur Anwendung bei der Behandlung von Krankheiten.

10. Verwendung von Verbindungen nach Anspruch 1 zur Herstellung von Arzneimitteln für die Behandlung von Atemwegserkrankungen.

## Claims

1. Compounds of the formula I, 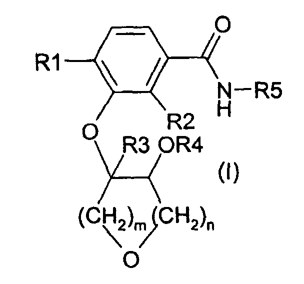 in which
R1 is 1-6C-alkoxy, 3-7C-cycloalkoxy, 3-7C-cycloalkylmethoxy, benzyloxy or 1-4C-alkoxy which is completely or predominantly substituted by fluorine,
R2 is hydrogen and
R3 is hydrogen
or
R2 and R3 are together a methylene group,
R4 is hydrogen, 1-8C-alkyl, 1-6C-alkoxy-1-4C-alkyl, 1-6C-alkylthio-1-4-alkyl, 1-6C-alkylsulfinyl-1-4C-alkyl, 1-6C-alkylsulfonyl-1-4C-alkyl, 1-8C-alkylcarbonyl, 3-7C-cycloalkyl, 3-7C-cycloalkylmethyl. phenyl-1-4C-alkyl or 1-4C-alkyl which is completely or predominantly substituted by fluorine,
R5 is phenyl, pyridyl, phenyl substituted by R51, R52 and R53 or pyridyl substituted by R54, R55, R56 and R57, where
R51 is hydroxyl, halogen, cyano, carboxyl, trifluoromethyl, 1-4C-alkyl, 1-4C-alkoxy, 1-4C-alkoxycarbonyl, 1-4C-alkylcarbonyl, 1-4C-alkylcarbonyloxy, amino, mono- or di-1-4C-alkylamino or 1-4C-alkylcarbonylamino,
R52 is hydrogen, hydroxyl, halogen, amino, trifluoromethyl, 1-4C-alkyl or 1-4C-alkoxy,
R53 is hydrogen, halogen, 1-4C-alkyl or 1-4C-alkoxy,
R54 hydroxyl, halogen, cyano, carboxyl, 1-4C-alkyl, 1-4C-alkoxy, 1-4C-alkoxycarbonyl or amino,
R55 is hydrogen, halogen, amino or 1-4C-alkyl,
R56 is hydrogen or halogen and
R57 is hydrogen or halogen,
n is 1 or 2,
m is 1 or 2,
where the sum of m and n may only assume the values 2 or 3,
the salts of these compounds and the N-oxides of the pyridines and their salts.

2. Compounds of the formula I according to claim 1, in which
R1 is 1-4C-alkoxy, 3-5C-cycloalkoxy, 3-5C-cycloalkylmethoxy or 1-4C-alkoxy which is completely or predominantly substituted by fluorine,
R2 is hydrogen and
R3 is hydrogen,
or
R2 and R3 together are a methylene group,
R4 is hydrogen, 1-6C-alkyl, 1-4C-alkoxy-1-4C-alkyl, 1-4C-alkylthio-1-4C-alkyl, 1-6C-alkylcarbonyl, 3-7C-cycloalkyl, 3-7C-cycloalkylmethyl, phenyl-1-4C-alkyl or 1-4C-alkyl which is completely or predominantly substituted by fluorine,
R5 is phenyl, pyridyl, phenyl substituted by R51, R52 and R53 or pyridyl substituted by R54, R55, R56 and R57, where
R51 is halogen, cyano, carboxyl, 1-4C-alkyl, 1-4C-alkoxy or 1-4C-alkoxycarbonyl,
R52 is hydrogen, halogen, 1-4C-alkyl or 1-4C-alkoxy,
R53 is hydrogen, halogen, 1-4C-alkyl or 1-4C-alkoxy,
R54 is halogen or 1-4C-alkyl,
R55 is hydrogen or halogen,
R56 is hydrogen or halogen and
R57 is hydrogen or halogen,
n is 1 or 2,
m is 1 or 2,
where the sum of m and n may only assume the values 2 or 3,
the salts of these compounds and the N-oxides of the pyridines and their salts.

3. Compounds of the formula I according to claim 1, in which
R1 is methoxy or difluoromethoxy,
R2 is hydrogen and
R3 is hydrogen
or
R2 and R3 together are a methylene group,
R4 is hydrogen, 1-6C-alkyl, 1-4C-alkoxy-1-4C-alkyl, 1-4C-alkylthio-1-4C-alkyl, 1-4C-alkylcarbonyl, phenethyl or benzyl,
R5 is 2-bromophenyl, 2-chlorophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2-chloro-6-fluorophenyl, 2,6-difluorophenyl, 2,6-dichlorophenyl, 3,5-dichloropyrid-4-yl, 3-methylpyrid-2-yl, 2-chloropyrid-3-yl, 3-chloropyrid-4-yl, 3,5-dibromopyrid-2-yl, 3,5-difluoropyrid-4-yl, 2,3,5,6-tetrafluoropyrid-4-yl, 3-chloro-2,5,6-trifluoropyrid-4-yl, 3,5-dichloro-2,6-difluoropyrid-4-yl or 2,6-dichloropyrid-3-yl,
n is 1,
m is 1 and
the salts of these compounds and the N-oxides of the pyridines and their salts.

4. Compounds of the formula I according to claim 1, in which
R1 is methoxy or difluoromethoxy,
R2 is hydrogen and
R3 is hydrogen,
or
R2 and R3 together are a methylene group,
R4 is hydrogen, 1-4C-alkyl, 1-2C-alkoxy-1-2C-alkyl, 1-2C-alkylthio-1-2C-alkyl, 1-4C-alkylcarbonyl or benzyl,
R5 is 3,5-dichloropyrid-4-yl, 2-chloropyrid-3-yl, 3-chloropyrid-4-yl, 3,5-dibromopyrid-2-yl, 3,5-difluoropyrid-4-yl, 2,3,5,6-tetrafluoropyrid-4-yl, 3-chioro-2,5,6-trifluoropyrid-4-yl, 3,5-dichloro-2,6-difluoropyrid-4-yl or 2,6-dichloropyrid-3-yl,
n is 1,
m is 1 and
the salts of these compounds and the N-oxides of the pyridines and their salts.

5. Compounds of the formula I according to claim 1, in which
R1 is methoxy,
R2 is hydrogen and
R3 is hydrogen,
or
R2 and R3 together are a methylene group,
R4 is hydrogen, methyl, ethyl, methylthiomethyl or benzyl,
R5 is 3,5-dichloropyrid-4-yl,
n is 1,
m is 1 and
the salts of these compounds.

6. Compounds according to claim 1, 2, 3, 4 or 5, in which R2 and R3 each have the meaning hydrogen.

7. Compounds according to claim 1, 2, 3, 4 or 5, in which R2 and R3 together are a methylene group.

8. Medicaments comprising at least one compound as claimed in claim 1 together with usual pharmaceutical auxiliaries and/or excipients.

9. Compounds of formula I for use in the treatment of diseases.

10. Use of compounds as claimed in claim 1 for the production of medicaments for the treatment of airway disorders.

## Revendications

1. Composés de formule I 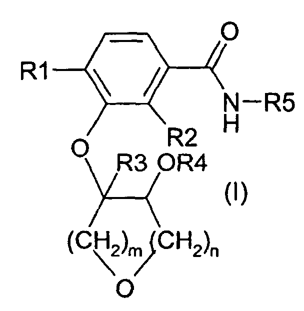 dans laquelle
R1 représente un groupe alcoxy en C₁₋₆, cycloalcoxy en C₃₋₇, (cycloalkyle en C₃₋₇)-méthoxy, benzyloxy ou alcoxy en C₁₋₄ totalement ou majoritairement fluoré,
R2 représente un atome d'hydrogène,
R3 représente un atome d'hydrogène,
ou
R2 et R3 représentent, conjointement, un groupe méthylène,
R4 représente un atome d'hydrogène, un groupe alkyle en C₁₋₈, (alcoxy en C₁₋₆)-(alkyle en C₁₋₄), (alkyle en C₁₋₆)-thio-(alkyle en C₁₋₄), (alkyle en C₁₋₆)-sulfinyl-(alkyle en C₁₋₄), (alkyle en C₁₋ ₆)-sulfonyl-(alkyle en C₁₋₄), (alkyle en C₁₋₈)-carbonyle, cycloalkyle en C₃₋₇, (cycloalkyle en C₃₋₇)-méthyle, phényl(alkyle en C₁₋₄) ou alkyle en C₁₋₄ totalement ou majoritairement fluoré,
R5 représente un groupe phényle, pyridyle, phényle portant des substituants R51, R52 et R53 ou pyridyle portant des substituants R54, R55, R56 et R57,
où
R51 représente un groupe hydroxy, halogéno, cyano, carboxyle, trifluorométhyle, alkyle en C₁₋₄, alcoxy en C₁₋₄, (alcoxy en C₁₋₄)-carbonyle, (alkyle en C₁₋₄)-carbonyle, (alkyle en C₁₋₄)-carbonyloxy, amino, mono- ou di(alkyle en C₁₋₄)amino ou (alkyle en C₁₋₄)-carbonylamino,
R52 représente un atome d'hydrogène, un groupe hydroxy, halogéno, amino, trifluorométhyle, alkyle en C₁₋₄ ou alcoxy en C₁₋₄,
R53 représente un atome d'hydrogène ou d'halogène, un groupe alkyle en C₁₋₄ ou alcoxy en C_{1-4,}
R54 représente un groupe hydroxyle, halogéno, cyano, carboxyle, alkyle en C₁₋₄, alcoxy en C₁₋₄, (alcoxy en C₁₋₄)-carbonyle ou amino,
R55 représente un atome d'hydrogène ou d'halogène, un groupe amino ou alkyle en C₁₋₄,
R56 représente un atome d'hydrogène ou d'halogène, et
R57 représente un atome d'hydrogène ou d'halogène,
n vaut 1 ou 2,
m vaut 1 ou 2,
la somme de m et n étant égale à 2 ou 3,
ainsi que les sels de ces composés et les N-oxydes des pyridines et les sels de ceux-ci.

2. Composés de formule (I) selon la revendication 1 dans lesquels
R1 représente un groupe alcoxy en C₁₋₄, cycloalcoxy en C₃₋₅, (cycloalkyle en C₃₋₅)-méthoxy ou alcoxy en C₁₋₄ totalement ou majoritairement fluoré,
R2 représente un atome d'hydrogène,
R3 représente un atome d'hydrogène,
ou
R2 et R3 représentent, conjointement, un groupe méthylène,
R4 représente un atome d'hydrogène, un groupe alkyle en C₁₋₆, (alcoxy en C₁₋₄)-(alkyle en C₁₋₄), (alkyle en C₁₋₄)-thio-(alkyle en C₁₋₄), (alkyle en C₁₋₆)-carbonyle, cycloalkyle en C₃₋₇, (cycloalkyle en C₃₋₇)-méthyle, phényl(alkyle en C₁₋₄) ou alkyle en C₁₋₄ totalement ou majoritairement fluoré,
R5 représente un groupe phényle, pyridyle, phényle portant des substituants R51, R52 et R53 ou pyridyle portant des substituants R54, R55, R56 et R57,
où
R51 représente un atome d'halogène, un groupe cyano, carboxyle, alkyle en C₁₋₄, alcoxy en C₁₋₄ ou (alcoxy en C₁₋₄)-carbonyle
R52 représente un atome d'hydrogène ou d'halogène, un groupe alkyle en C₁₋₄ ou alcoxy en C₁₋₄,
R53 représente un atome d'hydrogène ou d'halogène, un groupe alkyle en C₁₋₄ ou alcoxy en C₁₋₄,
R54 représente un atome d'halogène ou un groupe alkyle en C₁₋₄,
R55 représente un atome d'hydrogène ou d'halogène,
R56 représente un atome d'hydrogène ou d'halogène, et
R57 représente un atome d'hydrogène ou d'halogène,
n vaut 1 ou 2,
m vaut 1 ou 2,
la somme de m et n étant égale à 2 ou 3,
ainsi que les sels de ces composés et les N-oxydes des pyridines et les sels de ceux-ci.

3. Composés de formule (I) selon la revendication 1, dans lesquels
R1 représente un groupe méthoxy ou difluorométhoxy,
R2 représente un atome d'hydrogène et
R3 représente un atome d'hydrogène
ou
R2 et R3 représentent, conjointement, un groupe méthylène,
R4 représente un atome d'hydrogène, un groupe alkyle en C₁₋₆, (alcoxy en C₁₋₄)-(alkyle en C₁₋₄), (alkyle en C₁₋₄)-thio-(alkyle en C₁₋₄), (alkyle en C₁₋₄)-carbonyle, phénéthyle ou benzyle,
R5 représente un groupe 2-bromophényle, 2-chlorophényle, 2,6-dichloro-4-éthoxycarbonylphényle, 2,6-diméthoxyphényle, 4-cyano-2-fluorophényle, 2,4,6-trifluorophényle, 2-chloro-6-méthylphényle, 2,6-diméthylphényle, 2-chloro-6-fluorophényle, 2,6-difluorophényle, 2,6-dichlorophényle, 3,5-dichloropyrid-4-yle, 3-méthylpyrid-2-yle, 2-chloropyrid-3-yle, 3-chloropyrid-4-yle, 3,5-dibromopyrid-2-yle, 3,5-difluoropyrid-4-yle, 2,3,5,6-tétrafluoropyrid-4-yle, 3-chloro-2,5,6-trifluoropyrid-4-yle, 3,5-dichloro-2,6-difluoropyid-4-yle ou 2,6-dichloropyrid-3-yle,
n vaut 1,
m vaut 1,
ainsi que les sels de ces composés et les N-oxydes des pyridines et les sels de ceux-ci.

4. Composés de formule (I) selon la revendication 1, dans lesquels
R1 représente un groupe méthoxy ou difluorométhoxy,
R2 représente un atome d'hydrogène et
R3 représente un atome d'hydrogène
ou
R2 et R3 représentent, conjointement, un groupe méthylène,
R4 représente un atome d'hydrogène, un groupe alkyle en C₁₋₄, (alcoxy en C₁₋₂)-(alkyle en C₁₋₂), (alkyle en C₁₋₂)-thio-(alkyle en C₁₋₂), (alkyle en C₁₋₄)-carbonyle ou benzyle,
R5 représente un groupe 3,5-dichloropyrid-4-yle, 2-chloropyrid-3-yle, 3-chloropyrid-4-yle, 3,5-dibromopyrid-2-yle, 3,5-difluoropyrid-4-yle, 2,3,5,6-tétrafluoropyrid-4-yle, 3-chloro-2,5,6-trifluoropyrid-4-yle, 3,5-dichloro-2,6-difluoropyid-4-yle ou 2,6-dichloropyrid-3-yle,
n vaut 1,
m vaut 1,
ainsi que les sels de ces composés et les N-oxydes des pyridines et les sels de ceux-ci.

5. Composés de formule (I) selon la revendication 1, dans lesquels
R1 représente un groupe méthoxy,
R2 représente un atome d'hydrogène et
R3 représente un atome d'hydrogène,
ou
R2 et R3 représentent, conjointement, un groupe méthylène,
R4 représente un atome d'hydrogène, un groupe méthyle, éthyle, méthylthiométhyle ou benzyle,
R5 représente un groupe 3,5-dichloropyridyle,
n vaut 1 et m vaut 1,
ainsi que les sels de ces composés.

6. Composés selon les revendications 1, 2, 3, 4 ou 5 dans lesquels R2 et R3 représentent chacun un atome d'hydrogène.

7. Composés selon les revendications 1, 2, 3, 4 ou 5 dans lesquels R2 et R3 représentent conjointement un groupe méthylène.

8. Médicament contenant un ou plusieurs des composés selon la revendication 1, et des additifs et/ou excipients pharmaceutiques usuels.

9. Composés selon la revendication 1 destinés à être utilisés pour le traitement de maladies.

10. Utilisation de composés selon la revendication 1 pour la fabrication de médicaments destinés au traitement de maladies des voies respiratoires.
